# EUROPEAN PATENT SPECIFICATION

(11) **EP 3 556 329 B1**
(45) Date of publication and mention of the grant of the patent: **09.10.2024**
(21) Application number: 19153407.2
(22) Date of filing: 24.01.2019
(51) Int. Cl.: A61F 6/04, A61F 6/06

(54) **LUBRICIOUS CONDOM**
GLEITENDES KONDOM
PRÉSERVATIF LUBRIFIANT

(30) Priority: 20.04.2018 CN 201810358479
(43) Date of publication of application: 23.10.2019
(73) Proprietor: Wang, Hao, Chengdu Sichuan 610101 (CN)
(72) Inventor: Zhu, Deqi, Chengdu, Sichuan 610041 (CN); Wang, Hao, Chengu, Sichuan 610041 (CN); Liu, Xiaoqin, Chengu, Sichuan 610041 (CN)
(74) Representative: Wang, Bo

(56) References cited:
- WO-A1-2006/037321
- WO-A1-2016/137864
- WO-A1-89/01324

## Description

### Field of Invention

The present invention relates to condoms, specifically it is a condom with hydrophilic surface modification, which becomes lubricious when contacted with liquid or body fluids.

### Background

A condom is a sheath-shaped barrier device, used during sexual intercourse to reduce the probability of pregnancy or a sexually transmitted infection (STI). With proper use and use at every act of intercourse women whose partners use male condoms experience a 2% per-year pregnancy rate. With typical use the rate of pregnancy is 18% per-year. Their use greatly decreases the risk of gonorrhea, chlamydia, trichomoniasis, hepatitis B, and HIV/AIDS. To a lesser extent they also protect against genital herpes, human papillomavirus (HPV), and syphilis.

There are both male and female condoms. Male condoms are typically made from latex and less commonly from polyurethane or lamb intestine. Female condoms are typically made from polyurethane and may be used multiple times. But condoms made from other materials, such as animal intestines, are also available.

Condoms as a method of preventing STIs are on the World Health Organization's List of Essential Medicines, the most effective and safe medicines needed in a health system. Although condoms are proven to be an effective way to prevent STIs and pregnancy, many people are reluctant to use them because they are uncomfortable to use, with bad smells, or reduce pleasure. Globally less than 10% of those using birth control are using the condom. Rates of condom use are higher in the developed world. In United Kingdom the condom is the second most common method of birth control (22%) while in the United States it is the third most common (15%).

Many methods are tried to improve the user experience with condoms, for example, patent us9387120 disclosed a condom together with a lubrication composition, the lubricating composition containing a minimal amount of free water, and comprising one or more glycols, such that the glycols in the lubricant release heat and warmth when contact with compositions containing free water.

US patent application 20160043066A1 disclosed methods comprising the use of a macroinitiator and application protocols to apply a hydrophilic coating to latex, or natural rubber, and compositions resulting from such methods. The above-mentioned hydrophilic coating comprises a macroinitiating co-polymer and a hydrophilic polymer that form an interpenetrating co-polymer network on the surface of the latex article, which results in an increased sense of lubrication when in contact with water or an aqueous solution.

US patent application 20140326250 disclosed a condom which comprises, on one or more surfaces thereof, a self-lubricating coating comprising a dry powder having a particle size of 300 microns or less. The self-lubricating coating becomes lubricious when the coating comes into contact with a liquid environment. A method of making a self-lubricating condom comprises providing a dry condom and coating said condom, on one or more surfaces thereof, with a self-lubricating coating comprising a dry powder. The invention also provides the use of a dry powder such as xanthan gum to provide a self-lubricating coating for a condom.

WIPO Patent WO/2014/135855A1 disclosed a condom having a coating or deposit of a composition which includes a physiologically-active agent such as glyceryl trinitrate, wherein the condom has been treated with a neutralising or acidic material such that the condom, when immersed in water, results in the water having a pH of 7 or less. The condom may be treated with an acidic slurry of dusting powder. Also provided was a process for the manufacture of condoms, in which the process includes the steps of: 1) treating the formed condoms with a neutralising or acidic material; and 2)thereafter applying to the condoms a composition including a physiologically active agent such as glyceryl trinitrate, wherein the condoms, when immersed in water, results in the water having a pH of 7 or less.

US patent 8770201 B2 disclosed a condom with multifunctional coating. Condoms having an elastomeric layer and coating containing an anhydrous warming lubricant and at least one or more ingredients dispersed therein, the coating being disposed on one or both surfaces are provided. The ingredients disposed on the elastomeric layer can be solid particles or encapsulated ingredients that are insoluble in the anhydrous warming lubricant and soluble in water. Methods of producing and using these condoms are also provided.

US patent 8651110B2/8443809B2 disclosed a lubricated condom, including a non-porous sheath member, having an open end and a rounded end, and shaped to cover the glans and body portion of a penis, and at least one rib having at least one outer portion and at least one inner portion, wherein the at least one outer portion is attachedly fixed to said non-porous sheath member, and wherein an inner cylinder formed by at the at least one inner portion is exclusive from said non-porous sheath member, and including at least one lubricant within the inner cylinder and at least one hole extending from the inner cylinder through the outer portion to provide for flow of the lubricant outwardly from the inner cylinder through the at least one hole pursuant to application of a predetermined pressure, wherein the at least one rib extends at least one of substantially from the open end to the closed end and substantially about a circumference of the non-porous sheath member.

European Patent application EP0292220 disclosed a condom having a coating of thick material comprising a polysaccharide selected from the group consisting of dextran sulfate, pharmaceutically acceptable salts of dextran sulfate, hyaluronic acid and pharmaceutically acceptable salts of hyaluronic acid.

US patent application 20130269708 disclosed a penis enhanced condom, which is constructed with a basic latex sheath, and least partially covered by a sponge-like material for moisture absorption. The sponge-like material is covered by a smooth latex layer having multiple apertures for moisture migration.

US patent application 20120240937A1 disclosed an invention relating to novel articles, particularly latex barrier articles, being coated with an antimicrobial coating useful to reduce the risk of cross-contamination and/or infection by harmful pathogenic microorganisms.

US patent 8236370B2 disclosed packaged condoms that enable the use of a large quantity of a composition inside the condom. In addition, a sufficient quantity of certain spermicidal and/or microbicidal compositions is provided for efficient inactivation of sperm and pathogens. The disclosure also provides condoms with reduced tendency to slip or break. Compositions associated with different surfaces of the condom can be effectively segregated to their intended condom surface according to the present disclosure.

US patent application 20120121838A1 disclosed an elastomeric article with improved lubricity and donnablity and reduced stickiness/tackiness. According to the methods of the invention, the internal surface of the elastomeric article is coated with a polyisoprene coating. The coating of the invention is formed from synthetic polyisoprene rubber that may or may not contain minor amounts of other components. The coating is preferably directly bonded to the underlying elastomeric article.

US patent application 20050076917A1 disclosed a rolled-up lubricated condom formed by depositing a water-soluble lubricant to the rim of a rolled up condom after a surface of the condom has been coated with a surfactant modified powder. What were disclosed also include surfactant modified powder, methods of making surfactant modified powder, and methods of preparing a condom with surfactant modified powder.

US patent application 20060081264A1 disclosed a self-lubricating condom for use in mucosal areas and is provided to enhance sexual performance as well as keeping the condom moist and lubricated during sexual intercourse. The invention can be applied to condoms that are configured to cover the penis as well as the female cervix. The condom contains pockets of lubrication made from latex which wear due to friction and releases lubrication at different intervals due to the wear of the plastic of different thinness and thickness, moisture upon impact with the methacrylic acid co-polymer method or moisture upon impact with the gelatin method.

In general, three methods are used to improve the lubricity and usability: (1) adding lubricants; (2) using new condom materials, mostly latex with new additives; and (3) surface coating. Lubricants are not an ideal solution since they can damage the condoms, are uncomfortable to use, and their residues can be a health hazard when left in human's body. And new condom materials, which might show some benefits to certain degree, in general are not as good as the traditional latex or polyurethane materials as a whole. For surface coatings, they are either not lubricious enough, or cannot bond well with substrate material, or too thick to use. In summary, it still remains as a challenge to make condoms with acceptable lubricity and usability. Making condoms more comfortable to use, to lower the rate of STDs, is very meaningful for the users and the whole society.
WO 2006/037321 A1 relates to a medical device having a wetted hydrophilic coating comprising: a coating composition containing a hydrophilic polymer and a wetting agent comprising water and one or more lubricant(s). WO 89/01324 A1 relates generally to a natural feeling condom. WO 2016/137864 A1 discloses a composition comprising: a latex article having at least one layer of a hydrophilic coating, wherein the hydrophilic coating comprises a macroinitiating co-polymer and a hydrophilic polymer that form an interpenetrating co-polymer network on the surface of the latex article.

### Summary of the invention

To solve the problems of the current condoms on the market, the present invention discloses a safe, stable, lubricious, and strongly-bonded hydrophilic modification on the condom's surface, to make them more comfortable and better to use.

A zwitterion is a molecule with two or more functional groups, of which at least one has a positive and one has a negative electrical charge and the net charge of the entire molecule is zero. In most cases, these functional groups are not on neighboring atoms. Depending on its molecular structure, typical zwitterion includes phosphorylcholine, sulfobetaine, carboxybetaine, and mixed zwitterions, such as polypeptide, polybetaine, and polytrimethylamine oxide. Many zwitterions have superb hydrophilicity, outstanding thermal and chemical stability, and excellent biocompatibility. Because of their superhydrophilicity, if those zwitterion materials can be grafted onto the surface of condoms, they can form a thin but stable water film when contacted with liquids or body fluids, to greatly improve the surface lubricity without the need of lubricants. Meanwhile, because of the chemical bonding between substrate material and surface zwitterion modification, there will be very little if any zwitterion materials detached from the surface during use, which makes them an ideal material to make condoms better and safer.

### 1. Condom substrate materials

Condoms are typically made from latex, polyurethane, or lamb intestine. But condoms made from other materials, such as polyisoprene, silicone, and nitriles, are also available. The present invention can be applied onto these substrate materials, as well as other materials, such as polyamide, polyanhydride, polyazine, poly(carbonate), polyester, polyether, polyetheretherketone, polyguanidine, polyimide, polyketal, polyketone, polyolefin, polyorthoester, polyphosphazine, polysaccharide, polysiloxane, polysulfone, polyurea, polyurethane, halogenated polymer, epoxy resin, phenolic resin, or a copolymer or blend thereof.

### 2. Monomers

The monomers used in the present invention have a structure as the following:

B-L-Z

in which B might contain unsaturated double bonds, which can homo-polymerize, react with other monomers, or react with the substrate materials. Specifically, B can be chosen from: 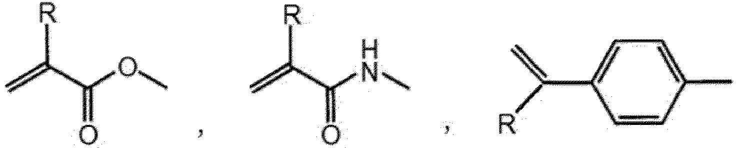
R is hydrogen, alkyl or substituted alkyl.
L doesn't exist, or is linear or branch alkyl, with one or more oxygen atoms;
Z is a zwitterion, which can be chosen from:

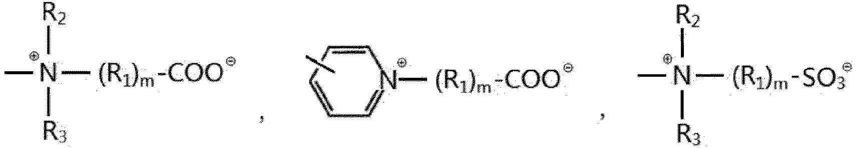

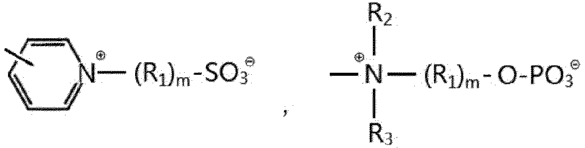

R1 is hydrogen, alkyl, or substituted alkyl.

R2 and R3 are independent, and are hydrogen, alkyl, or substituted alkyl.

m is integer from 1 to 7.

One or more than one monomers can be used during the reaction, to form alternating copolymer, segmented copolymer with pre-determined ratio, random copolymer, or homopolymer.

### 3. Grafting methods

Grafting reaction is needed to chemically bond the zwitterions used in present invention with condom substrate material. In general, almost all polymerization methods can be used to graft zwitterions to material surface, so for the present invention, the grafting methods can be used include but not limited to: Atom transfer radical polymerization (ATRP), ring-opening metathesis polymerization (ROMP), ultraviolet (UV) free radical polymerization, heat free radical polymerization, reduction-oxidation (redox) free radical polymerization, anionic or cationic polymerization, ring-opening polymerization, nitroxide-mediated radical polymerization, reversible addition-fragmentation chain-transfer polymerization (RAFT),telluride-medicated polymerization, and acyclic diene metathesis polymerization.

### (1) ATRP

Atom transfer radical polymerization (ATRP) is a means of forming a carbon-carbon bond with a transition metal catalyst. In ATRP reaction, free radicals are the active species, and the atom transfer step is crucial for uniform polymer chain growth.

In an ATRP process, the number of polymer chains is determined by the number of initiators. The most often used ATRP initiators include but not limited to alkyl halides, benzylic halides, α -bromo ester, α-halogenated ketone, α-halogenated nitrile, aryl sulfonyl chloride, azodiisobutyronitrile. In present invention alkyl halides, especially chloroalkanes and bromoalkanes, are preferably used as the initiator.

ATRP usually employs a transition metal complex as the catalyst, such as Cu, Fe, Ru, Ni, and Os. In an ATRP process, the dormant species is activated by the transition metal complex to generate radicals via one electron transfer process. Simultaneously the transition metal is oxidized to higher oxidation state. This reversible process rapidly establishes an equilibrium that is predominately shifted to the side with very low radical concentrations. In present invention, copper salts, especially copper chloride and copper bromide, are preferably used as the catalyst.

### (2) UV free radical polymerization

UV free radical polymerization can be initialized with UV initiator, which can be introduced into the condom substrate by mixing, imbibing or other methods. Condoms loaded with the initiator can then been put into the solution with zwitterion monomers, and after shedding with UV light, the grafting reaction can happen on the condom surface. Normally used UV initiators include but not limited to diphenyl(2,4,6-trimethylbenzoyl)phosphine oxide, ethyl (2,4,6-trimethylbenzoyl) phenylphosphinate, 2-methyl-4-(methylthio)-2-morpholinopropiophenone, 2-isopropyl thioxanthone, ethyl 4-dimethylaminobenzoate, 1-hydroxycyclohexyl phenyl ketone, 2-hydroxy-2-methylpropiophenone, methyl 2-benzoylbenzoate, benzophenone and derivatives.

### (3) Heat free radical polymerization

Heat free radical polymerization can be initialized by adding thermal initiators into the condom substrate material and then heating to generate heat free radicals. Considering the possible effects on condom substrate materials, the heating temperature is in general lower than 100°C, preferably lower than 80°C. Normally used thermal initiators include but not limited to: dicumyl peroxide, potassium peroxydisulfate, peroxyacetic acid, tert-butyl peroxyacetate, tert-butyl hydroperoxide, cumene hydroperoxide, 1,1-di(tert-butylperoxy)cyclohexane, 2,2'-azobis(isobutyronitrile), 4,4'-azobis(4-cyanovaleric acid).

### (4) Redox free radical polymerization

Redox initiators can be introduced into condom substrates, and free radicals, generated via reduction-oxidation reaction, can then initiate the polymerization process. Redox initiators in general include both oxidants and reductants. One advantage of redox polymerization is its fast initiation rate and mild polymerization conditions, which in general doesn't require the same high temperature as in the thermal free radical polymerization. Normally used oxidants include but not limited to hydrogen peroxide, persulfates, hydroperoxides, pyrosulfates, pyrophosphates, permanganates, manganese(III) salts, ceric salts, ferric salts, cyclohexanone peroxide, methyl ethyl ketone peroxide, and benzoyl peroxide. Normally used reductants include but not limited to ferrous salts, chromous salts, cupric salts, titanous salts, thiols, sulfates, and bisulfates.

### Examples

### Example 1 Lubricious condoms by ATRP method

Step 1:100 gram of N,N-dimethylaminoethyl methacrylate was dissolved into 1000 ml of glacial acetic acid. 40 gram of ethenesulfonyl chloride was slowly added into the solution, which was then stirred at room temperature for 24 hours. The precipitate was collected, washed in anhydrous ethanol twice, and then ground into powder after drying.

Step 2: Polyurethane condoms were first treated with chlorine plasma, and then added into 100 ml of 1:1 (v:v) methanol aqueous solution, which contained of 10 mM of CuCl₂, 20 mM of N, N, N', N', N"-pentamethyldiethylenetriamine, and 10% (w/v) of the product from Step 1. After sealing, samples and solution were purged with nitrogen for 15 minutes and then heated to 60 °C. After 3 hours, the condoms were taken out, first washed with the mixture of methanol and water, then washed with physiological saline solution, and dried in air.

### Example 2: Lubricious condoms by UV free radical polymerization

Step 1:100 gram of N,N-dimethylaminoethyl methacrylate was dissolved into 1000 ml of acetonitrile. Then 80 gram of 1,4-butanesultone and 300 mg of 1,3-dinitrobenzene were slowly added to the solution, which was refluxed at room temperature for 24 hours. The precipitate was collected, washed twice in acetonitrile, and dried at room temperature.

Step 2: Latex condoms were washed and cleaned, then immersed in 100 ml of 0.1M benzophenone in ethanol for 60 minutes. After drying in air, samples were put in 100 ml of 10% (w/v) solution of the product from Step 1 in water, which was then purged with nitrogen for 15 minutes and reacted in UV rotation reactor for 6 hours. Condoms were then taken out, rinsed with saline, and dried in air.

### Example 3: Lubricious condoms by heat free radical polymerization

Step 1: 100 gram of N,N-dimethylaminoethyl methacrylate was dissolved into 600 ml of anhydrous acetone. 55 gram of β-propiolactone was slowly added into the solution and then reacted under nitrogen at 15 °C for 6 hours. The precipitate was collected, washed with anhydrous acetone twice, dried, and then ground into powder.

Step 2: Latex condoms were first immersed into 100 ml of 1% (w/v) azobisisobutyronitrile in ethanol for 60 minutes, dried in air, and then put into 100 ml of 10% (w/v) solution of product from Step 1 and 1 mM of FeCl₂. After purging with nitrogen for 15 minutes, the solution was heated to 80 °C and reacted for 3 hours. Then the condoms were taken out, washed with saline, and dried.

### Example 4: Lubricous condom by redox free radical polymerization

Step 1: 100 gram of N,N-dimethylaminoethyl methacrylate was added into 400 ml of anhydrous acetone, and 75 gram of 1,3-propanesultone was dissolved into 100 ml of anhydrous acetone. The two solutions were slowly mixed together, stirred at room temperature for 4 hours, and left at room temperature for 7 days. Then the precipitate was collected, washed with anhydrous acetone and dried.

Step 2: Latex condoms were immersed in 100 ml of 1% (w/v) tert-butyl hydroperoxide in methanol for 60 minutes, dried in air, and then put into 100 ml of 10/% (w/v) solution of the product from Step 1 and 1 mg/ml of diammonium cerium(IV) nitrate in water. After purging with nitrogen for 15 minutes, the solution was heated to 60 °C and reacted for 3 hours. Then the condoms were taken out, washed with saline and water, and dried.

### Example 5: Mechanical testing for lubricious condoms

Condoms were tested following ISO 4074:2015 "natural rubber latex male condoms -- requirements and test methods", which includes the size, thickness, pinholes, aging, and strength. Compared to unmodified controls, lubricious condoms had thickness increase less than 0.5 µm, and there was no statistically significant change for other parameters.

### Example 6: The measurement of surface friction coefficient

To measure the surface friction coefficient of control and modified condoms, ASTM standard D1894 - 14 "Standard Test Method for Static and Kinetic Coefficients of Friction of Plastic Film and Sheeting" was referenced with modification. In details, condoms were put on stainless steel mandrels, kept flat, and contacted with the mandrel surface as much as possible. Then condoms together with the mandrels were put into saline, perpendicularly mounted, and a 2 N force was applied through a par of forceps. The friction coefficient measuring machine has a range from 0 to 5 N, with an accuracy higher than 0.2%. When the samples moved in a rate of 150 mm/min, the kinetic friction coefficient was measured. The results showed that control samples had friction coefficient higher than 0.5, while that of the modified ones lower than 0.05.

### Example 7: The stability of condom surface modification

ASTM D6279-15 "Standard Test Method for Rub Abrasion Mar Resistance of High Gloss Coatings" was referenced for the condom surface modification stability testing. In details, condoms were mounted on stainless mandrels, immersed into silane, hold with hands, and moved hands up and down with a relatively constant rate about 15 cm/min. One back and forth is a testing circle and 1000 such circles were tested, and then surface friction coefficient was measured again following the steps in example 6. It has been found that the surface friction coefficient didn't have any statistically significant change, which proved that the surface modification was mechanically stable.

### Example 8: Aging of modified condoms

Modified condoms were sealed and then kept at 70°C for 7 days. Then the surface friction coefficient was measured following the steps in example 6. It has been found that the surface friction coefficient didn't have any statistically significant change, which met the aging requirement for ISO 4074:2015 "Natural rubber latex male condoms -- Requirements and test methods".

## Claims

1. Condoms with surface hydrophilic and lubricous modifications, **characterized in that** said condoms are grafted with zwitterions which form lubricious water film in liquid environment to lower the surface friction.

2. The condoms of claim 1, where the monomers of zwitterions have the following structure:
B-L-Z
in which B was chosen from: 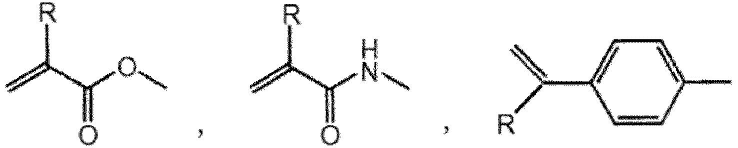 Wherein:
R is hydrogen, alkyl or substituted alkyl.
L doesn't exist, or is linear or branch alkyl, with one or more oxygen atoms;
Z is a zwitterion, which can be chosen from: 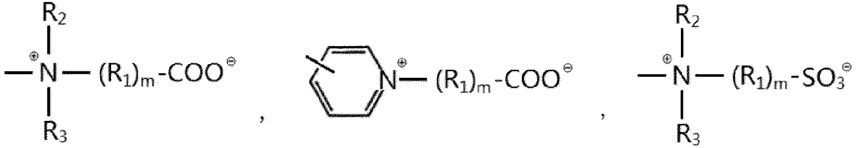 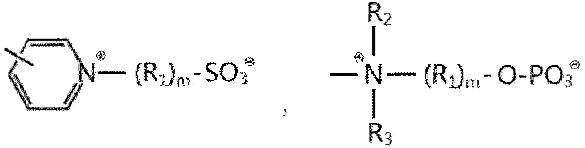
R1 is hydrogen, alkyl, or substituted alkyl.
R2 and R3 are independent, and are hydrogen, alkyl, or substituted alkyl.
m is integer from 1 to 7.

3. The condoms in claim 2, where the zwitterions include one or more monomers.

4. The condoms in claim 2, where the zwitterions are phosphocholines.

5. The condoms in claim 2, where the zwitterions are betaines.

6. The condoms in claim 5, where the zwitterions are carboxybetaines.

7. The condoms in claim 5, where the zwitterions are sulfobetaines.

8. The condoms in claim 2, where the condom substrate material is latex.

9. The condoms in claim 2, where the condom substrate material is polyurethane.

10. The condoms in claim 2, where the condoms are made from natural materials such as animal intestines.

## Patentansprüche

1. Kondome mit hydrophilen und gleitfähigen Oberflächenmodifikationen, **dadurch gekennzeichnet, dass** die Kondome mit Zwitterionen gepfropft sind, die in flüssiger Umgebung einen gleitfähigen Wasserfilm bilden, um die Oberflächenreibung zu verringern.

2. Kondome nach Anspruch 1, wobei die Monomere von Zwitterionen die folgende Struktur aufweisen:
B-L-Z
wobei B ausgewählt wurde aus: 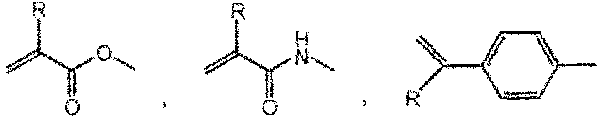 wobei:
R Wasserstoff, Alkyl oder substituiertes Alkyl ist;
L nicht existiert oder lineares oder verzweigtes Alkyl mit einem oder mehreren Sauerstoffatomen ist;
Z ein Zwitterion ist, das ausgewählt werden kann aus: 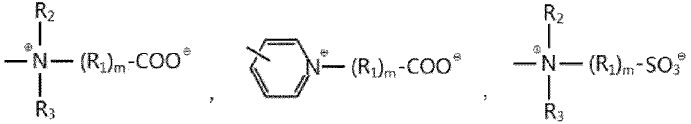 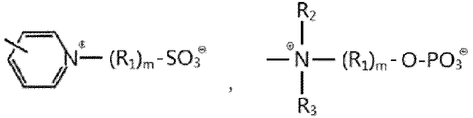
R1 Wasserstoff, Alkyl oder substituiertes Alkyl ist;
R2 und R3 unabhängig sind und Wasserstoff, Alkyl oder substituiertes Alkyl sind;
m eine ganze Zahl von 1 bis 7 ist.

3. Kondome nach Anspruch 2, wobei die Zwitterionen ein oder mehrere Monomere beinhalten.

4. Kondome nach Anspruch 2, wobei die Zwitterionen Phosphocholine sind.

5. Kondome nach Anspruch 2, wobei die Zwitterionen Betaine sind.

6. Kondome nach Anspruch 5, wobei die Zwitterionen Carboxybetaine sind.

7. Kondome nach Anspruch 5, wobei die Zwitterionen Sulfobetaine sind.

8. Kondome nach Anspruch 2, wobei das Kondomträgermaterial Latex ist.

9. Kondome nach Anspruch 2, wobei das Kondomträgermaterial Polyurethan ist.

10. Kondome nach Anspruch 2, wobei die Kondome aus natürlichen Materialien wie Tierdärmen hergestellt sind.

## Revendications

1. Préservatifs présentant des modifications superficielles hydrophiles et lubrifiantes, **caractérisés en ce que** lesdits préservatifs sont greffés avec des zwitterions qui forment un film d'eau lubrifiant dans un environnement liquide pour réduire le frottement superficiel.

2. Préservatifs selon la revendication 1, dans lesquels les monomères de zwitterions présentent la structure suivante :
B-L-Z
dans lequel B a été choisi parmi : 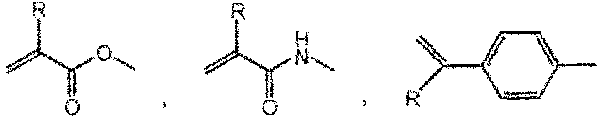 Où :
R est un hydrogène, un alkyle ou un alkyle substitué.
L n'existe pas, ou est un alkyle linéaire ou ramifié, avec un ou plusieurs atomes d'oxygène ;
Z est un zwitterion, qui peut être choisi parmi : 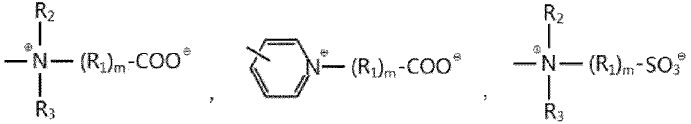 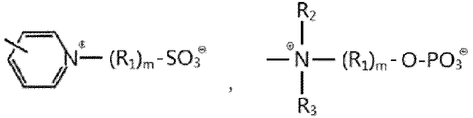
R1 est un hydrogène, un alkyle ou un alkyle substitué.
R2 et R3 sont indépendants et sont un hydrogène, un alkyle ou un alkyle substitué.
m est un nombre entier de 1 à 7.

3. Préservatifs selon la revendication 2, dans lesquels les zwitterions comprennent un ou plusieurs monomères.

4. Préservatifs selon la revendication 2, dans lesquels les zwitterions sont des phosphocholines.

5. Préservatifs selon la revendication 2, dans lesquels les zwitterions sont des bétaïnes.

6. Préservatifs selon la revendication 5, dans lesquels les zwitterions sont des carboxybétaïnes.

7. Préservatifs selon la revendication 5, dans lesquels les zwitterions sont des sulfobétaïnes.

8. Préservatifs selon la revendication 2, dans lesquels le matériau de substrat du préservatif est du latex.

9. Préservatifs selon la revendication 2, dans lesquels le matériau de substrat du préservatif est du polyuréthane.

10. Préservatifs selon la revendication 2, dans lesquels les préservatifs sont fabriqués à partir de matériaux naturels tels que des intestins d'animaux.
